(19) Europäisches Patentamt
European Patent Office
Office européen des brevets

(11) Veröffentlichungsnummer: **0 226 156 A2**

(12) **EUROPÄISCHE PATENTANMELDUNG**

(21) Anmeldenummer: 86117018.1

(22) Anmeldetag: 08.12.86

(51) Int.Cl.[4]: C 07 D 231/38
C 07 D 401/04, C 07 F 9/65
A 01 N 43/56, A 01 N 57/08
//C07D231/16, C07C109/04

(30) Priorität: 20.12.85 DE 3545347

(43) Veröffentlichungstag der Anmeldung: 24.06.87 Patentblatt 87/26

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Gehring, Reinhold, Dr.
Dasnöckel 49
D-5600 Wuppertal 11(DE)

(72) Erfinder: Lindig, Markus, Dr.
Dahlienweg 16
D-4010 Hilden(DE)

(72) Erfinder: Schallner, Otto, Dr.
Noldeweg 22
D-4019 Monheim(DE)

(72) Erfinder: Stetter, Jörg, Dr.
Gellertweg 4
D-5600 Wuppertal 1(DE)

(72) Erfinder: Santel, Hans-Joachim, Dr.
Grünstrasse 9a
D-5090 Leverkusen 1(DE)

(72) Erfinder: Schmidt, Robert R., Dr.
Im Waldwinkel 110
D-5060 Bergisch Gladbach 2(DE)

(72) Erfinder: Lürssen, Klaus, Dr.
August-Kierspel-Strasse 151
D-5060 Bergisch Gladbach 2(DE)

(54) **1-Aryl-pyrazole.**

(57) Die Erfindung betrifft neue 1-Aryl-pyrazole der allgemeinen Formel (I),

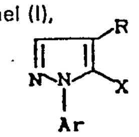

(I)

in welcher

R für Cyano oder Nitro steht,

Ar für jeweils gegebenenfalls substituiertes Phenyl oder Pyridyl steht und

X für eine Azidogruppe oder für einen Rest 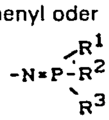

wobei

$R^1$, $R^2$ und $R^3$ unabhängig voneinander jeweils für Alkyl, Alkenyl, Alkinyl, Halogenalkyl, Alkoxy, Alkoxyalkyl, Cycloalkyl, Cycloalkyloxy oder für jeweils gegebenenfalls substituiertes Aryl, Aryloxy, Aralkyl oder Aralkyloxy stehen,

wobei jedoch für den Fall, daß R für Cyano steht und X gleichzeitig für eine Azidogruppe steht, Ar nicht für unsubstituiertes Phenyl oder für 5-Nitro-2-pyridyl steht, mehrere Verfahren zu ihrer Herstellung und ihre Verwendung als Herbizide und Wachstumsregulatoren.

EP 0 226 156 A2

Croydon Printing Company Ltd

BAYER AKTIENGESELLSCHAFT 5090 Leverkusen, Bayerwerk
Konzernverwaltung RP
Patentabteilung KM/m-c
Ib

## 1-Aryl-pyrazole

Die Erfindung betrifft neue 1-Aryl-pyrazole, mehrere Verfahren zu ihrer Herstellung und ihre Verwendung als Herbizide und Pflanzenwachstumsregulatoren.

Es ist bekannt, daß bestimmte 1-Aryl-pyrazole, wie beispielsweise das 4-Cyano-5-propionamido-1-(2,4,6-trichlorphenyl)-pyrazol herbizide Eigenschaften besitzen (vgl. DE-OS 3 226 513).

Die herbizide Wirksamkeit dieser vorbekannten Verbindungen gegenüber Unkräutern ist jedoch, ebenso wie ihre Verträglichkeit gegenüber wichtigen Kulturpflanzen nicht immer in allen Anwendungsbereichen völlig zufriedenstellend.

Weiterhin ist bekannt, daß die Verbindungen 5-Azido-4-cyano-1-phenyl-pyrazol und 5-Azido-4-cyano-1-(5-nitro-2-pyridyl)-pyrazol als Zwischenprodukte beschrieben sind (vgl. J. Heterocycl. Chem. 17, 1603-1604 [1980] sowie Rev. Latinoam. Quim. 13, 100-102 [1982]). Über eine herbizide

oder pflanzenwachstumsregulatorische Wirksamkeit dieser vorbeschriebenen Verbindungen ist jedoch nichts bekannt.

Es wurden neue 1-Aryl-pyrazole der allgemeinen Formel (I),

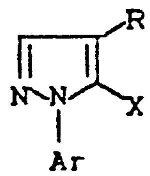

(I)

in welcher

R für Cyano oder Nitro steht,

Ar für jeweils gegebenenfalls substituiertes Phenyl oder Pyridyl steht und

X für eine Azidogruppe oder für einen Rest 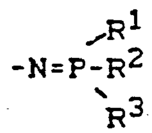

steht,

wobei

$R^1$, $R^2$ und $R^3$ unabhängig voneinander jeweils für Alkyl, Alkenyl, Alkinyl, Halogenalkyl, Alkoxy, Alkoxyalkyl, Cycloalkyl, Cycloalkyloxy oder für jeweils gegebenenfalls substituiertes Aryl, Aryloxy, Aralkyl oder Aralkyloxy stehen,

wobei jedoch für den Fall, daß R für Cyano steht und X gleichzeitig für eine Azidogruppe steht, Ar nicht für unsubstituiertes Phenyl oder für 5-Nitro-2-pyridyl steht,

gefunden.

Weiterhin wurde gefunden, daß man die neuen 1-Aryl-pyrazole der allgemeinen Formel (I),

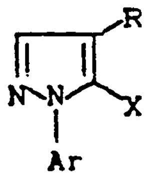 (I)

in welcher

R für Cyano oder Nitro steht,

Ar für jeweils gegebenenfalls substituiertes Phenyl oder Pyridyl steht und

X für eine Azidogruppe oder für einen Rest 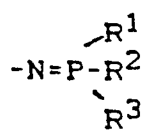 steht,

wobei

$R^1$, $R^2$ und $R^3$ unabhängig voneinander jeweils für Alkyl, Alkenyl, Alkinyl, Halogenalkyl, Alkoxyalkyl, Cycloalkyl, Alkoxy, Cycloalkyloxy oder für jeweils gegebenenfalls substituiertes Aryl, Aryloxy, Aralkyl oder Aralkyloxy stehen,

wobei jedoch für den Fall, daß R für Cyano steht und X gleichzeitig für eine Azidogruppe steht, Ar nicht für unsubstituiertes Phenyl oder für 5-Nitro-2-pyridyl steht, erhält,

wenn man

a-α) 5-Halogen-1-aryl-pyrazole der Formel (II),

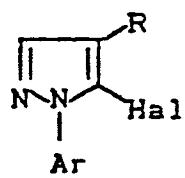

(II)

in welcher

R und Ar die oben angegebene Bedeutung haben und

Hal für Halogen steht,

mit Alkalimetallaziden der Formel (III),

$$M^{\ominus} - N_3^{\ominus} \quad (III)$$

in welcher

$M^{\ominus}$ für ein Alkalimetallkation steht,

gegebenenfalls in Gegenwart eines Verdünnungsmittels umsetzt,

oder wenn man

(a-ß) 5-Hydrazino-1-aryl-pyrazole der Formel (IV),

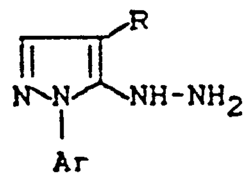

(IV)

in welcher

R und Ar die oben angegebene Bedeutung haben,

mit Alkalimetallnitriten der Formel (V)

$$M^{\oplus} - NO_2^{\ominus} \qquad (V)$$

in welcher

$M^{\oplus}$ für ein Alkalimetallkation steht,

in Gegenwart einer Katalysatorsäure und gegebenenfalls in Gegenwart eines Verdünnungsmittels diazotiert,

oder wenn man

b) die nach Verfahren (a-α) oder (a-ß) erhältlichen 5-Azido-1-arylpyrazole der Formel (Ia),

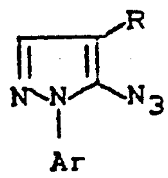

(Ia)

in welcher

R und Ar die oben angegebene Bedeutung haben,

in einer 2. Stufe mit Phosphinen bzw. Phosphiten der Formel (VI),

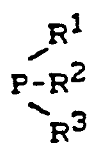

(VI)

in welcher

$R^1$, $R^2$ und $R^3$ die oben angegebene Bedeutung haben,

gegebenenfalls in Gegenwart eines Verdünnungsmittels umsetzt.

Schließlich wurde gefunden, daß die neuen 1-Aryl-pyrazole der allgemeinen Formel (I) herbizide, insbesondere auch selektiv-herbizide und pflanzenwuchsregulierende Eigenschaften besitzen.

Überraschenderweise zeigen die erfindungsgemäßen 1-Aryl-pyrazole der allgemeinen Formel (I) neben einer erheblich besseren allgemein-herbiziden Wirksamkeit gegenüber Unkräutern gleichzeitig eine erheblich höhere Kulturpflanzenverträglichkeit und darüber hinaus zusätzlich völlig überraschenderweise auch pflanzenwachstumsregulierende Eigenschaften im Vergleich zu den aus dem Stand der Technik bekannten 1-Aryl-pyrazolen, wie beispielsweise dem 4-Cyano-5-propionamido-1-(2,4,6-trichlorphenyl)-pyrazol, welche chemisch und wirkungsmäßig naheliegende Verbindungen sind.

Die erfindungsgemäßen 1-Aryl-pyrazole sind durch die Formel (I) allgemein definiert. Bevorzugt sind 1-Aryl-pyrazole der Formel (I), bei welchen

R für Cyano oder Nitro steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils bis zu 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder der Rest $-S(O)_n-R^4$,

X für eine Azidogruppe oder für einen Rest $-N{=}P(R^1)(R^2)(R^3)$ steht,

wobei

$R^1$, $R^2$ und $R^3$ unabhängig voneinander für jeweils geradkettiges oder verzweigtes Alkyl, Alkenyl, oder Alkinyl mit jeweils bis zu 8 Kohlenstoffatomen, für geradkettiges oder verzweigtes Halogenalkyl mit 1 bis 6 Kohlenstoffatomen und 1 bis 13 gleichen oder verschiedenen Halogenatomen, für geradkettiges oder verzweigtes Alkoxyalkyl mit jeweils 1 bis 4 Kohlenstoffatomen in den einzelnen Alkylteilen, für geradkettiges oder verzweigtes Alkoxy mit 1 bis 6 Kohlenstoffatomen, für Cycloalkyl oder Cycloalkoxy mit jeweils 3 bis 7 Kohlenstoffatomen oder für jeweils gegebenenfalls im Arylteil einfach oder mehrfach, gleich oder verschieden substituiertes Aralkyl, Aralkoxy, Aryl oder Aryloxy mit jeweils 6 bis 10 Kohlenstoffatomen in den einzelnen Arylteilen und gegebenenfalls ein bis drei Kohlenstoffatomen in den geradkettigen oder verzweigten Alkylteilen stehen, wobei als Arylsubstituenten jeweils infrage kommen: Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen,

$R^4$ für Amino, für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht,

wobei jedoch für den Fall, daß R für Cyano und X gleichzeitig für eine Azidogruppe steht, Ar nicht für unsubstituiertes Phenyl oder für 5-Nitro-2-pyridyl steht.

Besonders bevorzugt sind Verbindungen der Formel (I), bei welchen

R für Cyano oder Nitro steht,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder der Rest $-S(O)_n-R^4$ und

X für eine Azidogruppe oder für einen Rest $-N{=}P\begin{matrix}\diagup R^1\\ -R^2\\ \diagdown R^3\end{matrix}$ steht,

wobei

$R^1$, $R^2$ und $R^3$ unabhängig voneinander jeweils für Methyl, Ethyl, n- oder i-Propyl, n-, i-, s- oder t-Butyl, Allyl, Propargyl, Chlormethyl, Dichlormethyl, Trichlormethyl, Fluormethyl, Dichlorfluormethyl, Trifluormethyl, Dichlorfluormethyl, Difluorchlormethyl, Chlorethyl, Trichlorethyl, Pentachlorethyl, Trifluorethyl, Pentafluorethyl, Bromethyl, Methoxymethyl, Methoxyethyl, Ethoxymethyl, Ethoxyethyl, n-Propoxymethyl, i-Propoxymethyl, n-Propoxyethyl, i-Propoxyethyl, Methoxy, Ethoxy, n- oder i-Propoxy, n-, i-, s- oder t-Butoxy, Cyclopropyl, Cyclopentyl, Cyclohexyl, Cyclohexyloxy oder für jeweils gegebenenfalls ein- oder fünffach gleich oder verschieden substituiertes Benzyl, Benzyloxy, Phenyl oder Phenoxy stehen, wobei als Arylsubstituenten jeweils infrage kommen: Fluor, Chlor, Brom, Cyano, Nitro, Methyl, Ethyl, Methoxy, Methylthio oder Trifluormethyl,

$R^4$ für Amino, Methylamino, Ethylamino, Dimethylamino, Diethylamino, Fluordichlormethyl, Difluormethyl, Tetrafluorethyl, Trichlorethyl, Trifluormethyl, Methyl oder Ethyl steht

und

n für eine Zahl 0, 1 oder 2 steht,

wobei jedoch für den Fall, daß R für Cyano steht und X gleichzeitig für eine Azidogruppe steht, Ar nicht für unsubstituiertes Phenyl oder für 5-Nitro-2-pyridyl steht.

Ganz besonders bevorzugt sind Verbindungen der Formel (I),

bei welchen

R für Cyano oder Nitro steht,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder der Rest $-S(O)_n-R^4$ und

X für eine Azidogruppe oder für einen Rest $$-N=P\begin{matrix} / R^1 \\ -R^2 \\ \backslash R^3 \end{matrix}$$

steht,

wobei

$R^1$, $R^2$ und $R^3$ unabhängig voneinander jeweils für Methyl, Ethyl, n- oder i-Propyl, n-, i-, s- oder t-Butyl, Methoxy, Ethoxy, n- oder i-Propoxy, n-, i-, s- oder t-Butoxy oder für jeweils gegebenenfalls ein- bis fünffach gleich oder verschieden substituiertes Benzyl, Benzyloxy, Phenyl oder Phenoxy stehen, wobei als Arylsubstituenten jeweils infrage kommen: Fluor, Chlor, Brom, Cyano, Nitro, Methyl, Ethyl, Methoxy, Methylthio oder Trifluormethyl,

$R^4$ für Amino, Methylamino, Ethylamino, Dimethylamino, Diethylamino, Fluordichlormethyl, Difluormethyl, Tetrafluorethyl, Trichlorethyl, Trifluormethyl, Methyl oder Ethyl steht

und

n für eine Zahl 0, 1 oder 2 steht,

wobei jedoch für den Fall, daß R für Cyano steht und X gleichzeitig für eine Azidogruppe steht, Ar nicht für unsubstituiertes Phenyl oder für 5-Nitro-2-pyridyl steht.

Im einzelnen seien außer den bei den Herstellungsbeispielen genannten Verbindungen die folgenden substituierten 1-Aryl-pyrazole der allgemeinen Formel (I) genannt:

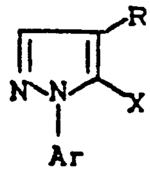 (I)

Tabelle 1

| R | X | Ar |
|---|---|---|
| CN | $N_3$ | 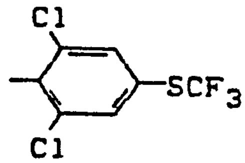 |
| $NO_2$ | $N_3$ | 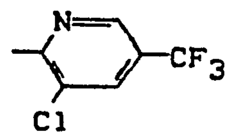 |
| $NO_2$ | $N_3$ | 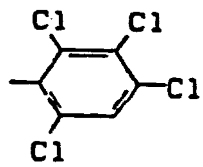 |
| CN | $OCH_3$<br>$-N=P{<}OCH_3$<br>$OCH_3$ | 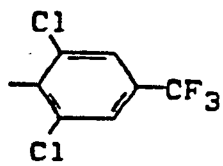 |
| $NO_2$ | $OCH_3$<br>$-N=P{<}OCH_3$<br>$OCH_3$ | 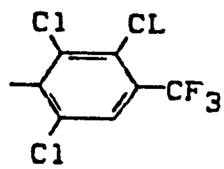 |
| $NO_2$ | $OC_2H_5$<br>$-N=P{<}OC_2H_5$<br>$OC_2H_5$ | 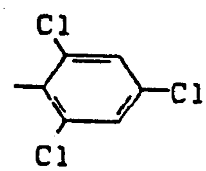 |
| $NO_2$ | $OC_2H_5$<br>$-N=P{<}OC_2H_5$<br>$OC_2H_5$ | 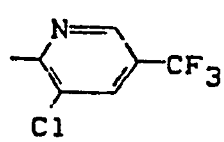 |

Tabelle 1 (Fortsetzung)

| R | X | Ar |
|---|---|---|
| CN | $OCH_3$<br>$-N=P<OCH_3$<br>$OCH_3$ | 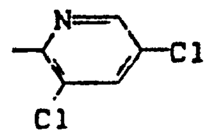 |
| $NO_2$ | $N_3$ | 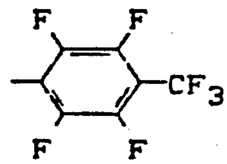 |
| $NO_2$ | $OCH_3$<br>$-N=P<OCH_3$<br>$OCH_3$ | 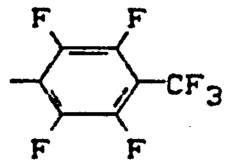 |
| $NO_2$ | $OC_2H_5$<br>$-N=P<OC_2H_5$<br>$CH_3$ | 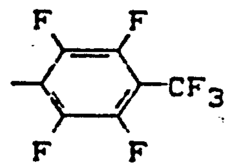 |
| CN | $-N=P(C_6H_5)_3$ | 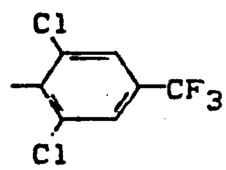 |
| $NO_2$ | $N_3$ | 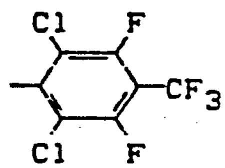 |
| $NO_2$ | $-N=P(OC_2H_5)_3$ | 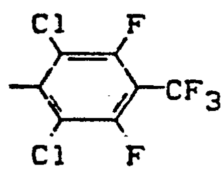 |
| $NO_2$ | $OC_2H_5$<br>$-N=P<CH_3$<br>$CH_3$ | 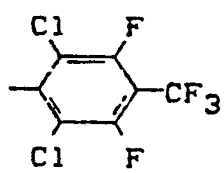 |

Tabelle 1 (Fortsetzung)

| R | X | Ar |
|---|---|---|
| $NO_2$ | $-N=P(C_6H_5)_3$ | 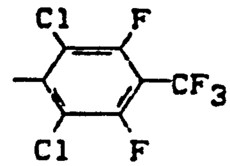 |
| CN | $OC_2H_5$<br>$-N=P{<}OC_2H_5$<br>$CH_3$ | 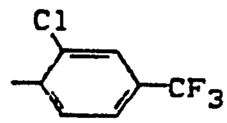 |
| CN | $OC_2H_5$<br>$-N=P{<}OC_2H_5$<br>$CH_3$ | 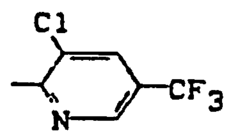 |

Verwendet man beispielsweise 5-Brom-4-nitro-1-(2,3,6-trichlor-4-trifluormethyl-phenyl)-pyrazol und Natriumazid als Ausgangsverbindungen, so läßt sich der Reaktionsablauf des erfindungsgemäßen Verfahrens (a-α) durch das folgende Formelschema darstellen:

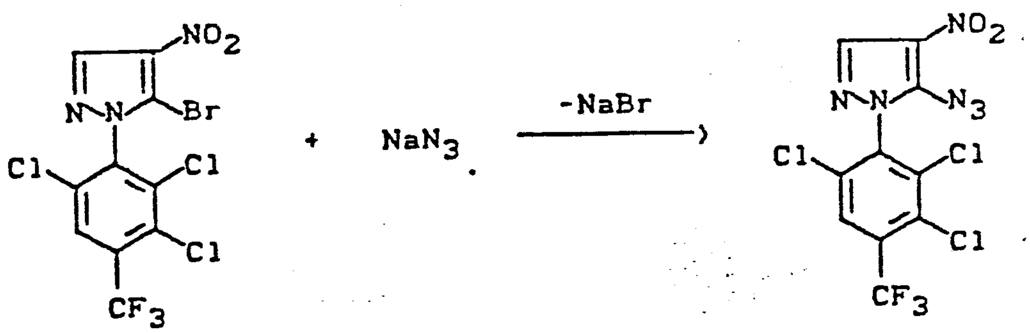


Verwendet man beispielsweise 4-Cyano-5-hydrazino-1-(2,4-dichlorphenyl)-pyrazol und Natriumnitrit als Ausgangsstoffe sowie Chlorwasserstoffsäure als Katalysator, so läßt sich der Reaktionsablauf des erfindungsgemäßen Verfahrens (a-ß) durch das folgende Formelschema darstellen:

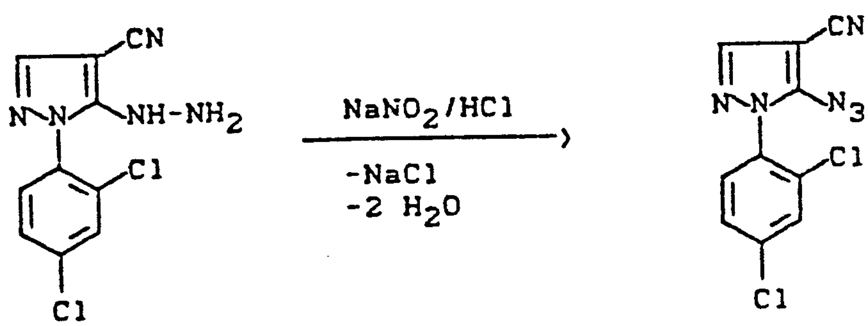

Verwendet man beispielsweise 5-Azido-4-cyano-1-(2,4-dichlorphenyl)-pyrazol und Triethylphosphit als Ausgangsstoffe, so läßt sich der Reaktionsablauf des erfindungsgemäßen Verfahrens (b) durch das folgende Formelschema darstellen:

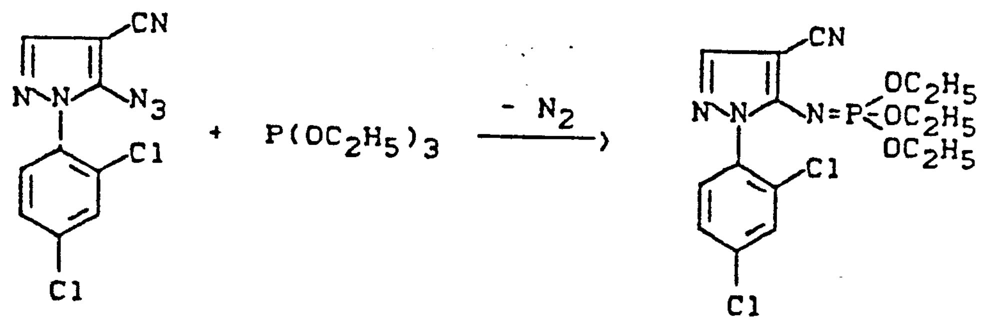

Die zur Durchführung des erfindungsgemäßen Verfahrens (a-α) als Ausgangsstoffe benötigten 5-Halogen-1-aryl-pyrazole sind durch die Formel (II) allgemein definiert. In dieser Formel (II) stehen R und Ar vorzugsweise für diejenigen Reste, die bereits im Zusammenhang mit der Beschreibung der erfindungsgemäßen Stoffe der Formel (I) als bevorzugt für diese Substituenten genannt wurden. Hal steht vorzugsweise für Chlor oder Brom.

Die 5-Halogen-1-aryl-pyrazole der Formel (II) sind noch nicht bekannt. Sie sind jedoch zum Teil Gegenstand eigener vorgängiger Patentanmeldungen (vgl. DE-P 3 501 323 vom 17.01.1985 und DE-P 3 520 329 vom 07.06.1985 und DE-P 3 520 330 vom 07.06.1985).

Man erhält sie beispielsweise, wenn man 5-Amino-1-aryl-pyrazole der Formel (VII),

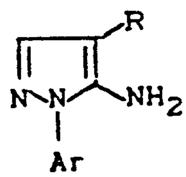

(VII)

in welcher

R und Ar die oben angegebene Bedeutung haben,

mit Nitritverbindungen der Formel (VIII),

$$R^5 - O - N = O \quad \text{(VIII)}$$

in welcher

$R^5$ für Wasserstoff, Alkyl oder für ein Alkalimetallkation steht,

in Gegenwart einer Halogenwasserstoffsäure wie beispielsweise Chlorwasserstoffsäure oder Bromwasserstoffsäure oder in Gegenwart eines Haloforms wie beispielsweise Chloroform oder Bromoform bei Temperaturen zwischen -20 °C und +80 °C in üblicher Art und Weise diazotiert (vgl. z.B. "Organikum" 15. Auflage VEB Deutscher Verlag der Wissenschaften, Berlin 1981 S. 652 ff; J. Chem. Soc. C, 1966, 1249 oder Rev. Latinoam. Quim. 13, 100-102 [1982]).

Die 5-Amino-1-aryl-pyrazole der Formel (VII) sind teilweise bekannt (vgl. z.B. EP 26 034, EP 53 678 oder EP 34 945, sowie DE-OS 3 402 308, DE-OS 3 226 496, DE-OS 3 408 727 oder DE-OS 3 420 985), teilweise sind sie Gegenstand eigener noch nicht veröffentlichter Patentanmeldungen (vgl. DE-P 3 520 330 vom 07.06.1985 und DE-P 3 520 327 vom 07.06.1985 und die Herstellungsbeispiele).

Man erhält sie beispielsweise, wenn man Aryl-hydrazine der Formel (IX),

$$Ar - NH - NH_2 \qquad (IX)$$

in welcher

Ar die oben angegebene Bedeutung hat,

(α) mit Acrylnitril-Derivaten der Formel (X),

$$Z - CH = C\Big\langle {CN \atop R} \qquad (X)$$

in welcher

R die oben angegebene Bedeutung hat und

Z für Halogen, Hydroxy, Alkoxy oder Dialkylamino steht,

oder

(β) mit 2-Halogenacrylnitrilen der Formel (XI),

$$CH_2 = C\begin{cases} CN \\ Hal^1 \end{cases} \qquad (XI)$$

in welcher

$Hal^1$ für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol, sowie gegebenenfalls in Gegenwart eines Reaktionshilfsmittels wie beispielsweise Natriumacetat bei Temperaturen zwischen -20 °C und +20 °C umsetzt zu den Arylhydrazin-Derivaten der Formel (XII),

$$Ar - NH - NH - CH = C\begin{cases} CN \\ R^6 \end{cases} \qquad (XII)$$

in welcher

Ar und $R^1$ die oben angegebene Bedeutung haben und

$R^6$ für Halogen, Cyano oder Nitro steht

und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50 °C und +150 °C cyclisiert, oder direkt in einem Reaktionsschritt, ohne Isolierung der Zwischenstufe der Formel (XII) gegebenenfalls in Gegenwart eines Verdünnungsmittels beispielsweise Ethylenglykolmonoethylether oder Ethanol bei Temperaturen zwischen +50 °C und +150 °C cyclisiert und gegebenenfalls die nach der Variante (β) erhältlichen in 4-Stellung unsubstituierten 5-Aminopyrazole der Formel (XIII),

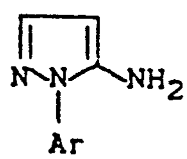

(XIII)

in welcher

Ar die oben angegebene Bedeutung hat,

in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20 °C und +50 °C nitriert.

Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung, zu schützen und die Aminoschutzgruppe nach erfolgter Nitrierung ebenfalls in üblicher Art und Weise beispielsweise durch Verseifung mit wässriger oder alkoholischer Base wieder abzuspalten.

Die Arylhydrazine der Formel (IX) sind bekannt (vgl. z.B. US-PS 4 127 575; US-PS 3 609 158; DE-OS 2 558 399; J. Chem. Soc. C, 1971, 167-174) oder können nach bekannten Verfahren in einfacher analoger Weise erhalten werden (vgl. z.B. Houben-Weyl "Methoden der organischen Chemie" Band X/2, S. 203, Thieme Verlag Stuttgart, 1967), indem man beispielsweise die bekannten Aniline bzw. Pyridylamine der Formel (XIV),

$$Ar - NH_2 \qquad (XIV)$$

in welcher

Ar die oben angegebene Bedeutung hat,

mit Natriumnitrit in Gegenwart einer Säure wie beispielsweise Schwefelsäure, und dann mit Zinn-II-chlorid ebenfalls in Gegenwart einer Säure wie beispielweise Salzsäure bei Temperaturen zwischen -20 °C und +80 °C umsetzt oder indem man Halogenaromate der Formel (XV),

$$Ar - Hal^2 \qquad (XV)$$

in welcher

Ar die oben angegebene Bedeutung hat und

$Hal^2$ für Halogen, insbesondere für Fluor, Chlor oder Brom steht,

mit Hydrazinhydrat gegebenenfalls in Gegenwart eines Verdünnungsmittels wie beispielsweise Pyridin oder Dioxan bei Temperaturen zwischen 0 °C und 150 °C umsetzt.

Die Nitritverbindungen der Formel (VIII), die Acrylnitril-Derivate der Formel (X), die 2-Halogenacrylnitrile der Formel (XI), die Aniline und Pyridylamine der Formel (XIV) und die Halogenaromate der Formel (XV) sind allgemein bekannte Verbindungen der organischen Chemie.

Die zur Durchführung des erfindungsgemäßen Verfahrens (a-α) weiterhin als Ausgangsstoffe benötigten Alkalimetallazide sind durch die Formel (III) allgemein definiert. In dieser Formel (III) steht $M^{\oplus}$ vorzugsweise für ein Natrium- oder Kaliumkation.

Die Alkalimetallazide der Formel (III) sind allgemein bekannte Verbindungen.

Die zur Durchführung des erfindungsgemäßen Verfahrens (a-β) als Ausgangsstoffe benötigten 5-Hydrazino-1-aryl-pyrazole sind durch die Formel (IV) allgemein definiert. In dieser Formel (IV) stehen R und Ar vorzugsweise für

diejenigen Reste, die bereits im Zusammenhang mit der Beschreibung der erfindungsgemäßen Stoffe der Formel (I) als bevorzugt für diese Substituenten genannt wurden.

Die 5-Hydrazino-1-aryl-pyrazole der Formel (IV) sind noch nicht bekannt. Sie sind jedoch Gegenstand einer eigenen vorgängigen Patentanmeldung (vgl. DE-P 3 528 478 vom 08.08.1985).

Man erhält sie, wenn man 5-Halogen-1-aryl-pyrazole der Formel (II),

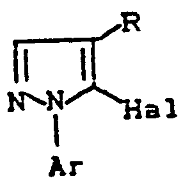

(II)

in welcher

R und Ar die oben angegebene Bedeutung haben und

Hal für Halogen, insbesondere für Chlor oder Brom steht,

mit Hydrazinhydrat gegebenenfalls in Gegenwart eines Verdünnungsmittels wie beispielsweise Dioxan bei Temperaturen zwischen 50 °C und 120 °C umsetzt.

Die zur Durchführung des erfindungsgemäßen Verfahrens (a-β) weiterhin als Ausgangsstoffe benötigten Alkalimetallnitrite sind durch die Formel (V) allgemein definiert.

In dieser Formel (V) steht $M^{\oplus}$ vorzugsweise für ein Natrium- oder Kaliumkation.

Die Alkalimetallnitrite der Formel (V) sind allgemein bekannte Verbindungen.

Die zur Durchführung des erfindungsgemäßen Verfahrens (b) als Ausgangsstoffe benötigten 5-Azido-1-aryl-pyrazole sind durch die Formel (Ia) allgemein definiert. In dieser Formel (Ia) stehen R und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Azido-1-aryl-pyrazole der Formel (Ia) sind erfindungsgemäße Verbindungen und erhältlich mit Hilfe der erfindungsgemäßen Verfahren (a-α) oder (a-β).

Die zur Durchführung des erfindungsgemäßen Verfahrens (b) weiterhin als Ausgangsstoffe benötigten Phosphine bzw. Phosphite sind durch die Formel (VI) allgemein definiert. In dieser Formel (VI) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Phosphine bzw. Phosphite der Formel (VI) sind allgemein bekannte Verbindungen der organischen Chemie.

Als Verdünnungsmittel zur Durchführung des erfindungsgemäßen Verfahrens (a-α) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Mit besonderem Vorzug verwendet man Dimethylsulfoxid als Verdünnungsmittel.

Die Reaktionstemperaturen können bei der Durchführung des erfindungsgemäßen Verfahrens (a-α) in einem größeren Bereich variiert werden. Im allgemeinen arbeitet man bei Temperaturen zwischen -20 °C und +80°C, vorzugsweise bei Temperaturen zwischen 0 °C und +50 °C.

Zur Durchführung des erfindungsgemäßen Verfahrens (a-α) setzt man pro Mol an 5-Halogen-1-aryl-pyrazol der Formel (II) im allgemeinen 1.0 bis 5.0 Mol, vorzugsweise 1.0 bis 2.0 Mol an Alkalimetallazid der Formel (III). Die Aufarbeitung, Isolierung und Charakterisierung der 5-Azido-1-aryl-pyrazole der Formel (Ia) erfolgt nach allgemein üblichen Verfahren.

0226156

Als Verdünnungsmittel zur Durchführung des erfindungsgemäßen Verfahrens (a-β) kommt hauptsächlich Wasser oder wässrige Säuren, wie beispielsweise wässrige Salzsäure infrage, welche in diesem Fall gleichzeitig als Verdünnungsmittel sowie als Katalysatorsäure eingesetzt werden.

Das erfindungsgemäße Verfahren (a-β) wird in Gegenwart einer anorganischen Säure als Katalysator durchgeführt. Als solche verwendet man vorzugsweise starke Mineralsäuren, mit besonderem Vorzug Chlorwasserstoffsäure.

Die Reaktionstemperaturen können bei der Durchführung des erfindungsgemäßen Verfahrens (a-β) in einem größeren Bereich variiert werden. Im allgemeinen arbeitet man bei Temperaturen zwischen -30 °C und +50 °C, vorzugsweise bei Temperaturen zwischen -20 °C und +30 °C.

Zur Durchführung des erfindungsgemäßen Verfahrens (a-β) setzt man pro Mol an 5-Hydrazino-1-aryl-pyrazol der Formel (IV) 1.0 bis 2.5 Mol, vorzugsweise 1.0 bis 1.5 Mol an Alkalimetallnitrit der Formel (V) ein. Die Reaktionsdurchführung, Aufarbeitung und Isolierung der 5-Azido-1-aryl-pyrazole der Formel (Ia) erfolgt nach allgemein üblichen Verfahren.

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +120 °C, vorzugsweise bei Temperaturen zwischen 0 °C und +50 °C.

Zur Durchführung des erfindungsgemäßen Verfahrens (b) setzt man pro Mol an 5-Azido-1-aryl-pyrazol der Formel (Ia) im allgemeinen 1.0 bis 2.0 Mol, vorzugsweise äquimolare Mengen an Phosphinen bzw. Phosphiten der Formel (VI) ein. Die Reaktionsdurchführung, Aufarbeitung und Isolierung der Reaktionsprodukte der Formel (I) erfolgt nach üblichen Verfahren.

Die erfindungsgemäßen Verbindungen der Formel (Ib),

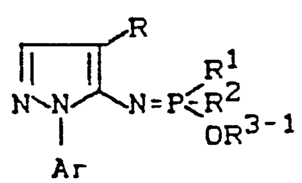

(Ib)

in welcher

R, $R^1$, $R^2$ und Ar die oben angegebene Bedeutung haben und

$R^{3-1}$ für Alkyl, Cycloalkyl, für gegebenenfalls substituiertes Aralkyl oder Aryl steht,

und welche erhältlich sind mit Hilfe des erfindungsgemäßen Verfahrens (b), sind außerdem geeignet als Zwischenprodukte zur Herstellung weiterer Herbizide, welche Gegenstand der eigenen vorgängigen Patentanmeldung DE-P 35 39 844 vom 09.11.1985 sind.

So erhält man Verbindungen der Formel (XVI),

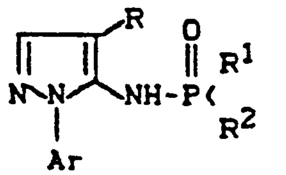

(XVI)

in welcher

R, $R^1$, $R^2$ und Ar die oben angegebene Bedeutung haben,

wenn man Verbindungen der Formel (Ib),

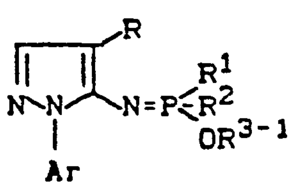

(Ib)

in welcher

R, $R^1$, $R^2$, $R^{3-1}$ und Ar die oben angegebene Bedeutung haben,

mit Wasser, gegebenenfalls in Gegenwart eines Verdünnungsmittels wie beispielsweise Xylol und gegebenenfalls in Gegenwart eines Katalysators, wie beispielsweise Benzoesäure bei Temperaturen zwischen 50 °C und 180 °C umsetzt (vgl. Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter, in monokotylen und dikotylen Kulturen wie beispielsweise Weizen, Gerste, Reis oder Baumwolle einsetzen.

Darüber hinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Aerosole, Suspensions-Emulsions-Konzentrate, Wirkstoff-imprägnierte Natur- und synthetische Stoffe, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie

Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethyl-phenyl)-harnstoff; N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5-(4H)-on; 2,4-Dichlorphenoxyessigsäure; 2,4-Dichlorphenoxy-propionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichlorpyrid-2-yloxy)-phenoxy]-propionsäure-2-ben-zyloxyethylester), -(trimethylsilylmethylester) oder -(2,2-diethoxyethylester); Methyl-5-(2,4-dichlorphen-oxy)-2-nitrobenzoat; 3,5-Diiod-4-hydroxybenzo nitril; 3-Isopropyl-2,1,3-benzothiadiazin-4-on-2,2-dioxid; 2-Chlor-N-{[(4-methoxy-6-methyl-1,3,5-triazin-2yl)-amino]-carbonyl}-benzolsulfonamid; 4-Ethylamino-2-t-butylamino-6-methylthio-s-triazin; 2-{4-[(3-Chlor-5-(trifluormethyl)-2-pyridyl)-oxy]-phenoxy}-propansäure bzw. propansäureethylester; 3,5-Dibrom-4-hydroxy-benzonitril; 2-[4-(2,4-Dichlorphenoxy)-phenoxy]-propionsäuremethylester; N-(1-Ethylpropyl)-3,4-dimethyl-2,6-dinitroanilin; N-Methyl-2-(1,3-benzthiazol-2-yloxy)-acetanilid; N,N-Diisopropyl-S-(2,3,3-trichlor-allyl)-thiolcarbamat sowie 1-Methyl-3-phenyl-5-(3-trifluormethyl-phenyl)-4-pyridon sind gegebenenfalls von Vorteil. Einige Mischungen zeigen überraschenderweise auch synergistische Wir-kung.

0226156

Auch eine Mischung mit anderen bekannten Wirkstoffen wie Fungiziden, Insektiziden, Akariziden, Nematiziden, Schutzstoffen gegen Vogelfraß, Pflanzennährstoffen und Bodenstrukturverbesserern ist möglich.

Die Wirkstoffe können als solche, in Form ihrer Formulierungen oder den daraus durch weiteres Verdünnen bereiteten Anwendungsformen wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Verwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

In entsprechenden Aufwandmengen zeigen die erfindungsgemäßen Wirkstoffe darüberhinaus auch eine blattinsektizide Wirksamkeit.

Die Herstellung und die Verwendung der erfindungsgemäßen Wirkstoffe geht aus den nachfolgenden Beispielen hervor:

Herstellungsbeispiele:

Beispiel 1:

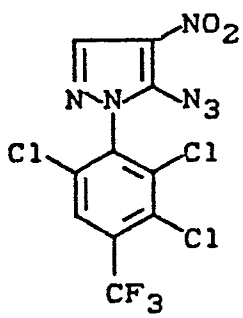

(Verfahren a-α)

Zu 3,0 g (0.007 Mol) 5-Brom-4-nitro-1-(2,3,6-trichlor-4-trifluormethyl-phenyl)-pyrazol in 50 ml Dimethylsulfoxid gibt man 1,0 g (0.015 Mol) Natriumazid, rührt 15 Stunden bei Raumtemperatur, gießt den Ansatz in 150 ml Wasser, extrahiert mehrfach mit Chloroform und Essigsäureethylester, trocknet die vereinigten organischen Phasen über Magnesiumsulfat, engt im Vakuum ein und reinigt das ölige Rohprodukt chromatographisch (Kieselgel; Laufmittel: Chloroform/Aceton 9:1). Man erhält 0,68 g (24 % der Theorie) an 5-Azido-4-nitro-1-(2,3,6-trichlor-4-trifluormethyl-phenyl)-pyrazol vom Schmelzpunkt 84 °C-88 °C.

Herstellung der Ausgangsverbindungen:

Beispiel II-1

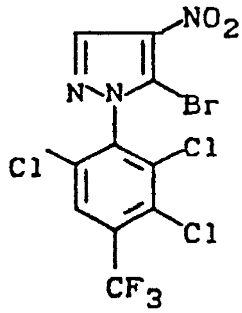

5,2 g (0.014 Mol) 5-Amino-4-nitro-1-(2,3,6-trichlor-4-trifluormethyl-phenyl)-pyrazol in 16 ml (0.18 Mol) Bromoform werden tropfenweise unter Rühren innerhalb von 10 Minuten mit 4,8 ml (0.04 Mol) t-Butyl-nitrit versetzt, wobei die Temperatur der Reaktionsmischung auf 50 °C ansteigt. Nach beendeter Zugabe rührt man eine weitere Stunde bei Rückflußtemperatur, engt die Mischung im Vakuum ein und reinigt das zurückbleibende Öl säulenchromatographisch (Kieselgel/Laufmittel: Chloroform/Aceton 9:1).

Man erhält 5,0 g (82 % der Theorie) an 5-Brom-4-nitro-1-(2,3,6-trichlor-4-trifluormethyl-phenyl)-pyrazol vom Schmelzpunkt 97 °C-99 °C.

Beispiel VII-1

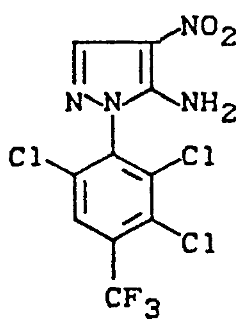

7,5 g (0.0227 Mol) 5-Amino-1-(2,3,6-trichlor-4-trifluormethyl-phenyl)-pyrazol und 2,2 ml (2,3 g = 0.023 Mol) Acetanhydrid in 20 ml Eisessig werden ca. 4 Stunden bei Raumtemperatur gerührt, bis im Dünnschichtchromatogramm kein Ausgangsprodukt mehr nachweisbar ist. Dann gibt man weitere 2,7 ml (2,9 g = 0.0288 Mol) Acetanhydrid und bei 5 °C bis 10 °C 1,2 ml (1,8 g = 0.028 Mol) 98-prozentige Salpetersäure zu, rührt 8 Stunden bei Raumtemperatur, entfernt das Lösungsmittel im Vakuum und nimmt den Rückstand in 30 ml Ethanol und 20 ml konzentrierter Salzsäure auf. Man erhitzt für 12 Stunden auf Rückflußtemperatur, engt im Vakuum ein, nimmt den Rückstand in 100 ml Dichlormethan auf, wäscht vorsichtig ($CO_2$-Entwicklung) mit 100 ml gesättigter Natriumhydrogencarbonatlösung, trocknet über Magnesiumsulfat und entfernt das Lösungsmittel im Vakuum.

Man erhält 7,5 g (84 % der Theorie) an 5-Amino-4-nitro-1-(2,3,6-trichlor-4-trifluormethyl-phenyl)-pyrazol vom Schmelzpunkt 78 °C bis 85 °C.

Beispiel XIII-1

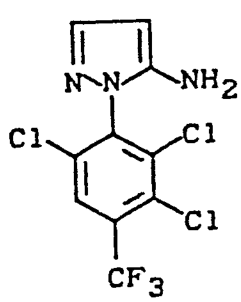

45 g (0.17 Mol) 2,3,6-Trichlor-4-trifluormethyl-phenylhydrazin und 35 mg (0.1 mMol) Ethylendiamintetraessigsäure-Dinatriumsalz (Titriplex III) in 260 ml Methanol werden bei Rückflußtemperatur tropfenweise innerhalb von 30 Minuten mit 57 ml (62,7 g = 0.71 Mol) 2-Chloracrylnitril versetzt und weitere 5 Stunden bei Rückflußtemperatur (ca. 65 °C) gerührt. Die Reaktionsmischung wird im Vakuum eingeengt, der Rückstand in 260 ml Methanol aufgenommen und bei Raumtemperatur tropfenweise unter Rühren innerhalb von 15 Minuten mit 15,4 ml (0.541 Mol) konzentrierter Schwefelsäure versetzt. Dabei steigt die Temperatur der Reaktionsmischung auf 32 °C. Nach beendeter Zugabe rührt man 30 Stunden bei 55 °C, kühlt auf Raumtemperatur ab, setzt 56 g (0.534 Mol) Natriumcarbonat zu, rührt abermals 4 Stunden bei Raumtemperatur, engt im Vakuum ein, nimmt den Rückstand in 550 ml Dichlormethan auf, gibt 55 ml Wasser zu, rührt 8 Stunden bei Raumtemperatur, trennt die organische Phase ab, wäscht mit 250 ml konzentrierter wässriger Natriumchloridlösung, trocknet über Magnesiumsulfat und entfernt das Lösungsmittel im Vakuum.

Man erhält 52,6 g (94 % der Theorie) am 5-Amino-1-(2,3,6-trichlor-4-trifluormethyl-phenyl)-pyrazol vom Schmelzpunkt 109 °C-114 °C.

Beispiel IX-1

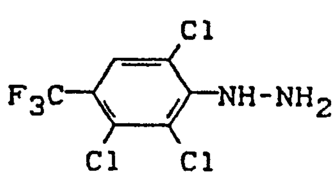

200 g (0.704 Mol) 1,2,3,4-Tetrachlor-5-trifluormethylbenzol und 240 ml (247,2 g/4.94 Mol) Hydrazinhydrat in 500 ml

Dioxan werden 14 Stunden lang unter Rückfluß erhitzt. Von der abgekühlten zweiphasigen Reaktionsmischung trennt man die schwerere (wässrige) Phase ab und engt die organische Phase im Vakuum zur Trockne ein. Der Rückstand wird in 600 ml Wasser und 100 ml Dichlormethan suspendiert, mit 10-prozentiger wässriger Natriumhydroxidlösung auf pH 10 eingestellt und langsam auf 30 °C bis 35 °C erwärmt, wobei sich aus der trüben Suspension zwei klare Phasen bilden. Man läßt auf Raumtemperatur abkühlen, trennt die organische Phase ab, wäscht sie mit 200 ml konzentrierter wässriger Natriumchloridlösung, trocknet über Magnesiumsulfat und entfernt das Lösungsmittel im Vakuum. Das Rohprodukt wird in siedendem Hexan 3 bis 4 Stunden gerührt, danach 15 Stunden bei 0 °C bis 5 °C gekühlt, kalt abgesaugt und 2 bis 3 Stunden bei 50 °C im Vakuum getrocknet.

Man erhält 146 g (71,2 % der Theorie) an 2,3,6-Trichlor-4-trifluormethyl-phenylhydrazin vom Schmelzpunkt 67 °C-70 °C mit einem gaschromatographisch bestimmten Gehalt von 96 %.

Beispiel 2:

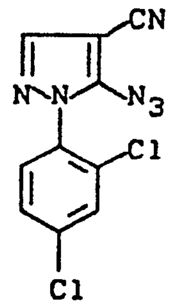

(Verfahren a-β)

Zu 2,7 g (0.01 Mol) 5-Hydrazino-4-cyano-1-(2,4-dichlorphenyl)-pyrazol in einer Lösung aus 1,9 ml konzentrierter Salzsäure in 10 ml Wasser gibt man bei 0 °C 10 ml Ether und anschließend 0,85 g (0.012 Mol) Natriumnitrit in 10 ml Wasser. Nach beendeter Zugabe fügt man weitere 20 ml Wasser und 30 ml Ether zu, filtriert, trennt die Etherphase ab, trocknet über Calciumchlorid, engt im Vakuum ein und reinigt das so erhältliche Öl chromatographisch (Kieselgel; Laufmittel: Dichlormethan/Aceton 9:1). Man erhält 1,2 g (43 % der Theorie) an 5-Azido-4-cyano-1-(2,4-dichlorphenyl)-pyrazol als Öl.

IR : $\nu$ = 2260 ($N_3$); 2190 (CN) $cm^{-1}$.

Herstellung der Ausgangsverbindungen

Beispiel IV-1

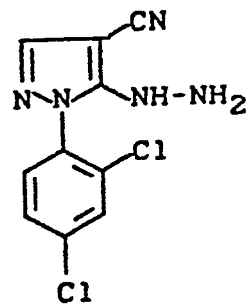

6,4 g (0.02 Mol) 5-Brom-4-cyano-1,(2,4-dichlorphenyl)-pyrazol und 15 g (0.2 Mol) Hydrazinhydrat in 80 ml Dioxan werden 20 Stunden bei Rückflußtemperatur gerührt, dann unter vermindertem Druck eingeengt, der Rückstand mit Wasser verrührt, abgesaugt und zweimal aus Ethanol umkristallisiert. Man erhält 2,1 g (40 % der Theorie) an 4-Cyano-5-hydrazino-1-(2,4-dichlorphenyl)-pyrazol vom Schmelzpunkt 199 °C-204 °C.

Beispiel II-2

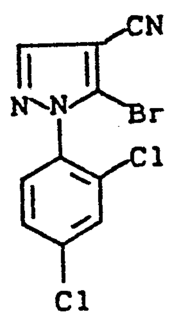

Zu einer Suspension aus 12,7 g (0.05 Mol) 5-Amino-4-cyano-1-(2,4-dichlorphenyl)-pyrazol in 100 ml Bromwasserstoffsäure gibt man bei -5 °C bis 0 °C 6 g (0.09 Mol) Natriumnitrit in 15 ml Wasser, rührt bis zum Ende der Gasentwicklung, wobei die Temperatur auf 30 °C ansteigt. Der feste Rückstand wird abgesaugt, in Wasser aufgeschlämmt, mit Natriumhydrogencarbonat neutralisiert, wieder abgesaugt und getrocknet.

Man erhält 14,5 g (91,5 % der Theorie) an 5-Brom-4-cyano-1-(2,4-dichlorphenyl)-pyrazol vom Schmelzpunkt 84 °C (Zers.).

Beispiel 3:

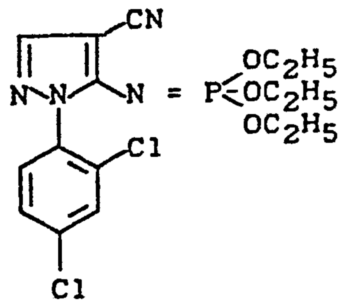

(Verfahren b)

Zu einer Lösung aus 2,2 g (0,013 Mol) Triethylphosphit in 20 ml Ether gibt man tropfenweise unter Rühren eine Lösung aus 3,7 g (0.013 Mol) 5-Azido-4-cyano-1-(2,4-dichlorphenyl)-pyrazol in 20 ml Ether. Zur Aufarbeitung wäscht man mit Wasser, trocknet über Natriumsulfat, entfernt das Lösungsmittel im Vakuum. Man erhält 2,7 g (50 % der Theorie) an 4-Cyano-5-[N-(triethoxyphosphorimido)]-1-(2,4-dichlorphenyl)-pyrazol vom Schmelzpunkt 50 °C.

Beispiel 4:

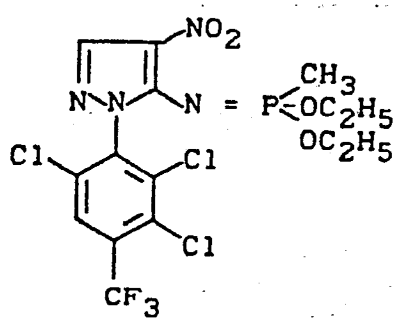

(Verfahren b)

Eine Lösung aus 4,5 g (0.011 Mol) 5-Azido-4-nitro-1-(2,3,6-trichlor-4-trifluormethyl-phenyl)-pyrazol in 20 ml Ether gibt man tropfenweise zu 1,7 g (0.012 Mol) Methylphosphonigsäurediethylester in 20 ml Ether. Zur Aufarbeitung entfernt man das Lösungsmittel im Vakuum und reinigt das zurückbleibende Öl chromatographisch (Kieselgel; Laufmittel: Dichlormethan). Man erhält 2,8 g (50 % der Theorie) an 4-Nitro-5-[N-(Diethoxymethyl-phosphorimido)]-1-(2,3,6-trichlor-4-trifluormethyl-phenyl)-pyrazol vom Schmelzpunkt 79 °C-83 °C.

In entsprechender Weise und gemäß den allgemeinen Angaben zur Herstellung erhält man die folgenden 1-Aryl-pyrazole der allgemeinen Formel (I):

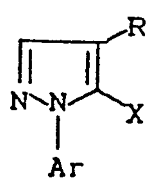

(I)

Tabelle 2

| Bsp. Nr. | R | X | Ar | Schmelzpunkt/°C |
|---|---|---|---|---|
| 5 | $NO_2$ | $N_3$ | 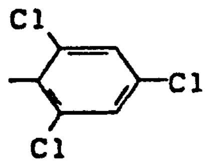 | 130 (Zers.) |
| 6 | CN | $N_3$ | 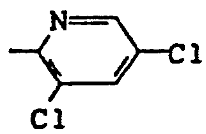 | 120 |
| 7 | $NO_2$ | $N_3$ | 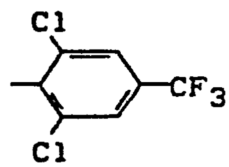 | 89 |
| 8 | CN | $N_3$ | 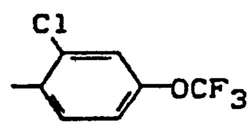 | $^1$H-NMR*) 8,5 |
| 9 | $NO_2$ | $N_3$ | 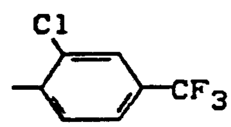 | 66 |
| 10 | $NO_2$ | $N_3$ | 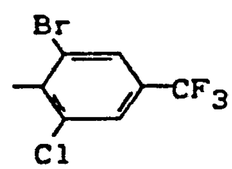 | 84-86 |
| 11 | $NO_2$ | $-N=P(OC_2H_5)_3$ | 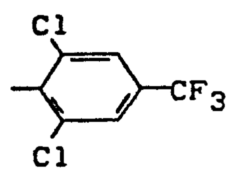 | 73 |
| 12 | CN | 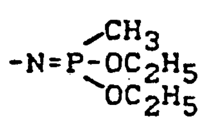 | 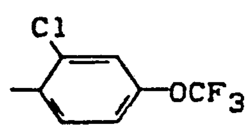 | $^1$H-NMR*): 1,7 |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Herstellung eines Folgeproduktes:

Beispiel XVI-1:

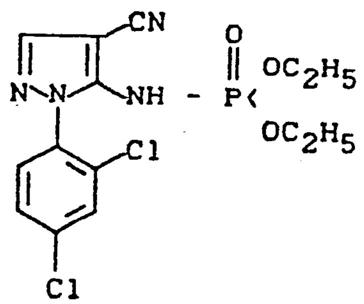

1,3 g (0.003 Mol) 4-Cyano-5-[N-(triethoxyphosphorimido)]-1-(2,4-dichlorphenyl)-pyrazol und 0,5 g (0.004 Mol) Benzoesäure werden in 20 ml Xylol mit wenig Wasser 3 Tage auf 150 °C erhitzt. Zur Aufarbeitung entfernt man das Lösungsmittel im Vakuum, nimmt den Rückstand in Dichlormethan auf, wäscht je zweimal mit wässriger Natriumbicarbonatlösung und Wasser, trocknet über Natriumsulfat, engt im Vakuum ein und reinigt den Rückstand chromatographisch (Kieselgel; Laufmittel: Dichlormethan/Aceton 9:1). Man erhält 0,4 g (35 % der Theorie) an 4-Cyano-5-[N-(O,O-Diethylphosphoryl)-amino]-1-(2,4-dichlorphenyl)-pyrazol vom Schmelzpunkt 70 °C.

Anwendungsbeispiele:

In den folgenden Anwendungsbeispielen wurde die nachstehend aufgeführte Verbindung als Vergleichsubstanz eingesetzt:

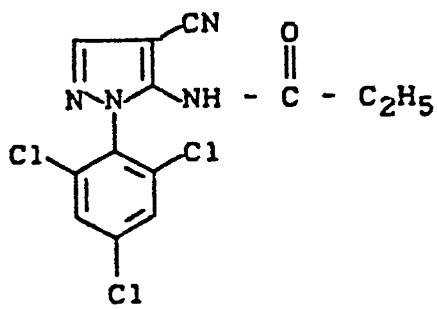

(A)

4-Cyano-5-propionamido-1-(2,4,6-trichlor-phenyl)-pyrazol (bekannt aus DE-OS 3 226 513).

0226156

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigt in diesem Test z.B. die Verbindung gemäß der Herstellungsbeispiele: (1), (4), (7) und (10).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z.B die Verbindungen gemäß der Herstellungsbeispiele: 1, 4 und 7.

## Beispiel C

### Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen boniert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

Eine deutliche Wirksamkeit zeigt in diesem Test beispielsweise die Verbindung gemäß dem Herstellungsbeispiel: 7

Patentansprüche

1. 1-Aryl-pyrazole der allgemeinen Formel (I),

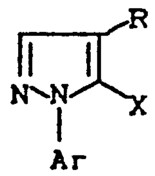

(I)

in welcher

R für Cyano oder Nitro steht,

Ar für jeweils gegebenenfalls substituiertes Phenyl oder Pyridyl steht und

X für eine Azidogruppe oder für einen Rest 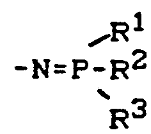 steht,

wobei

$R^1$, $R^2$ und $R^3$ unabhängig voneinander jeweils für Alkyl, Alkenyl, Alkinyl, Halogenalkyl, Alkoxy, Alkoxyalkyl, Cycloalkyl, Cycloalkyloxy oder für jeweils gegebenenfalls substituiertes Aryl, Aryloxy, Aralkyl oder Aralkyloxy stehen,

wobei jedoch für den Fall, daß R für Cyano steht und X gleichzeitig für eine Azidogruppe steht, Ar nicht für unsubstituiertes Phenyl oder für 5-Nitro-2-pyridyl steht.

2. 1-Aryl-pyrazole der Formel (I) gemäß Anspruch 1, in welcher

R für Cyano oder Nitro steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils bis zu 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder der Rest $-S(O)_n-R^4$,

X für eine Azidogruppe oder für einen Rest $-N=P(R^1)(R^2)(R^3)$ steht,

wobei

$R^1$, $R^2$ und $R^3$ unabhängig voneinander für jeweils geradkettiges oder verzweigtes Alkyl, Alkenyl, oder Alkinyl mit jeweils bis zu 8 Kohlenstoffatomen, für geradkettiges oder verzweigtes Halogenalkyl mit 1 bis 6 Kohlenstoffatomen und 1 bis 13 gleichen oder verschiedenen Halogenatomen, für geradkettiges oder verzweigtes Alkoxyalkyl mit jeweils 1 bis 4 Kohlenstoffatomen in den einzelnen Alkylteilen, für geradkettiges oder verzweigtes Alkoxy mit 1 bis 6 Kohlenstoffatomen, für Cycloalkyl oder Cycloalkoxy mit jeweils 3 bis 7 Kohlenstoffatomen oder für jeweils gegebenenfalls im Arylteil einfach oder mehrfach, gleich oder verschieden substituiertes Aralkyl, Aralkoxy, Aryl oder Aryloxy mit jeweils 6 bis 10 Kohlenstoffatomen in den einzelnen Arylteilen und gegebenenfalls ein bis drei Kohlenstoffatomen in den geradkettigen oder verzweigten Alkylteilen stehen, wobei als Arylsubstituenten jeweils infrage kommen: Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen,

$R^4$ für Amino, für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht,

wobei jedoch für den Fall, daß R für Cyano und X gleichzeitig für eine Azidogruppe steht, Ar nicht für unsubstituiertes Phenyl oder für 5-Nitro-2-pyridyl steht.

3. 1-Aryl-pyrazole der Formel (I) gemäß Anspruch 1, in welcher

R für Cyano oder Nitro steht,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder der Rest $-S(O)_n-R^4$ und

X für eine Azidogruppe oder für einen Rest 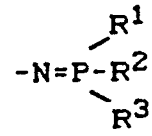 steht,

wobei

$R^1$, $R^2$ und $R^3$ unabhängig voneinander jeweils für Methyl, Ethyl, n- oder i-Propyl, n-, i-, s- oder t-Butyl, Allyl, Propargyl, Chlormethyl, Dichlormethyl, Trichlormethyl, Fluormethyl, Dichlorfluormethyl, Trifluormethyl, Dichlorfluormethyl, Difluorchlormethyl, Chlorethyl, Trichlorethyl, Pentachlorethyl, Trifluorethyl, Pentafluorethyl, Bromethyl, Methoxymethyl, Methoxyethyl, Ethoxymethyl, Ethoxyethyl, n-Propoxymethyl, i-Propoxymethyl, n-Propoxyethyl, i-Propoxyethyl, Methoxy, Ethoxy, n- oder i-Propoxy, n-, i-, s- oder t-Butoxy, Cyclopropyl, Cyclopentyl, Cyclohexyl, Cyclohexyloxy oder für jeweils gegebenenfalls ein- oder fünffach gleich oder verschieden substituiertes Benzyl, Benzyloxy, Phenyl oder Phenoxy stehen, wobei als Arylsubstituenten jeweils infrage kommen: Fluor, Chlor, Brom, Cyano, Nitro, Methyl, Ethyl, Methoxy, Methylthio oder Trifluormethyl,

$R^4$ für Amino, Methylamino, Ethylamino, Dimethylamino, Diethylamino, Fluordichlormethyl, Difluormethyl, Tetrafluorethyl, Trichlorethyl, Trifluormethyl, Methyl oder Ethyl steht

und

n für eine Zahl 0, 1 oder 2 steht,

wobei jedoch für den Fall, daß R für Cyano steht und X gleichzeitig für eine Azidogruppe steht, Ar nicht für unsubstituiertes Phenyl oder für 5-Nitro-2-pyridyl steht.

4. Verfahren zur Herstellung von 1-Aryl-pyrazolen der Formel (I),

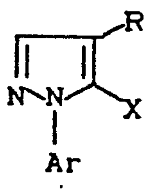

(I)

in welcher

R für Cyano oder Nitro steht,

Ar für jeweils gegebenenfalls substituiertes Phenyl oder Pyridyl steht und

X für eine Azidogruppe oder für einen Rest 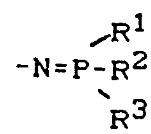

steht,

wobei

$R^1$, $R^2$ und $R^3$ unabhängig voneinander jeweils für Alkyl, Alkenyl, Alkinyl, Halogenalkyl, Alkoxy, Alkoxyalkyl, Cycloalkyl, Cycloalkyloxy oder für jeweils gegebenenfalls substituiertes Aryl, Aryloxy, Aralkyl oder Aralkyloxy stehen,

wobei jedoch für den Fall, daß R für Cyano steht und X gleichzeitig für eine Azidogruppe steht, Ar nicht für unsubstituiertes Phenyl oder für 5-Nitro-2-pyridyl steht.

dadurch gekennzeichnet, daß man

a-α) 5-Halogen-1-aryl-pyrazole der Formel (II),

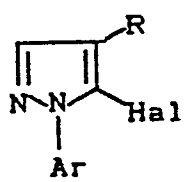

(II)

in welcher

R und Ar die oben angegebene Bedeutung haben und

Hal für Halogen steht,

mit Alkalimetallaziden der Formel (III),

$$M^{\oplus} - N_3^{\ominus} \qquad (III)$$

in welcher

$M^{\oplus}$ für ein Alkalimetallkation steht,

gegebenenfalls in Gegenwart eines Verdünnungsmittels umsetzt,

oder daß man

(a-β) 5-Hydrazino-1-aryl-pyrazole der Formel (IV),

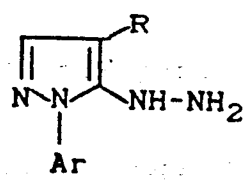

(IV)

in welcher

R und Ar die oben angegebene Bedeutung haben,

mit Alkalimetallnitriten der Formel (V)

$$M^{\oplus} - NO_2^{\ominus} \quad (V)$$

in welcher

$M^{\oplus}$ für ein Alkalimetallkation steht,

in Gegenwart einer Katalysatorsäure und gegebenenfalls in Gegenwart eines Verdünnungsmittels diazotiert,

oder daß man

b) die nach Verfahren (a-α) oder (a-β) erhältlichen 5-Azido-1-arylpyrazole der Formel (Ia),

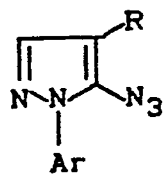

(Ia)

in welcher

R und Ar die oben angegebene Bedeutung haben,

in einer 2. Stufe mit Phosphinen bzw. Phosphiten der Formel (VI),

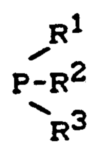

(VI)

in welcher

$R^1$, $R^2$ und $R^3$ die oben angegebene Bedeutung haben,

gegebenenfalls in Gegenwart eines Verdünnungsmittels umsetzt.

5. Herbizide und pflanzenwuchsregulierende Mittel, gekennzeichnet durch einen Gehalt an mindestens einem

1-Aryl-pyrazol der Formel (I) gemäß den Ansprüchen 1 bis 4.

6. Verfahren zur Bekämpfung von Unkräutern, dadurch gekennzeichnet, daß man 1-Aryl-pyrazole der Formel (I) gemäß den Ansprüchen 1 bis 4 auf die Unkräuter und/oder ihren Lebensraum einwirken läßt.

7. Verwendung von 1-Aryl-pyrazolen der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man 1-Aryl-pyrazole der Formel (I) gemäß den Ansprüchen 1 bis 4 mit Streckmitteln und/oder oberflächenaktiven Substanzen vermischt.